# EUROPEAN PATENT APPLICATION

(11) **EP 0 997 121 A1**
(43) Date of publication of application: **03.05.2000**
(21) Application number: 98830647.8
(22) Date of filing: 26.10.1998
(51) Int. Cl.: A61F 9/007

(54) **Tip for phacoemulsifier**

(71) Applicant: Optikon 2000 S.p.a., 00138 Roma (IT)
(72) Inventor: Crozafon, Philippe, 9800 Principato di Monaco (MC); Camerlengo, Flavio, Optikon 2000 S.p.A., 00138 Roma (IT)
(74) Representative: Iannone, Carlo Luigi

(57) **Abstract**

The present invention relates to an improved tip (1) for ophtalmic surgery, particularly for the excision of cataract, having a junction proximal end (2) and a distal end (4) contacting the crystalline during the surgical intervention, wherein said end (4) is subjected to antireflective treatment, and the portion (3) between the distal end (4) and the proximal end (2) is lined by PTFE (teflon), and the junction proximal end is subjecting to polishing.

## Description

The present invention concerns to an improved tip for surgery, particularly for oculistic surgery, and more particularly for the excision of cataract.

More particularly, the invention relates to a tip of the above kind for the cataract excision by ultrasound "phacoemulsifier".

In the following, the specification will be directed to the description of an instrument to be used in optical surgery, but it is intended that the inventive solution can be used for any surgery instrument.

As it is well known, "phacoemulsifier" is substantially an instrument comprised of a control unit and a "handpiece" or "handle" by which the surgeon carries out the surgical intervention.

A needle or tip is connected to the end of the handpiece, said needle or tip being oscillated with an ultrasonic frequency by a "piezoelectric" or "magnetostrictive" system housed within the same handpiece.

"Handpiece" is further provided with an infusion and suction system, the first one acting by gravity and the second one being operated by a vacuum pump.

The suction system is realised in such a way to act coaxially through the needle and is used to suck the crystalline (cataract) crushed by the vibratory motion of the needle.

Instead, the infusion system acts concentrically and outward with respect to the needle thus irrigating the eye by a physiological solution to restore the liquid sucked together with the crystalline.

Particularly, irrigation passes through the annular space created by a little sleeve, usually comprised of silicon, provided externally with respect to the needle.

The needle - sleeve system is introduced within the "front chamber" of the eye, by a corneal incision having dimensions proportional to the needle (2,5 - 3 mm).

One of the problems that could incur using said surgical technique concerns the more or less marked burning of the cornea on the two incision flaps though which the needle passes, with the heat generated by friction due to the rubbing of the needle against the silicon sleeve.

During the surgical intervention, the motions of the needle carried out by the surgeon and the two flaps exert on the sleeve radial centripetal forces putting the same in contact with the needle axially oscillating with an ultrasonic frequency.

During the contact between the oscillating needle and the silicon sleeve, a remarkable heating is generated also due to the fact that the irrigation of the physiological solution is locally interrupted.

The passage of the physiological solution normally flowing between needle and sleeve in any case exercises a cooling and lubricating action for the motion between needle and sleeve, so that even its temporary interruption is very critical.

In the art, solutions have been suggested, concerning the geometry of the sleeve, and substantially aiming to maintain and irrigation flow even during the contact between needle and sleeve.

As to the material by which the sleeve has been realised, alternative solutions to the use of the broadly employed silicon rubber have been suggested.

For example, low friction coefficient materials (PTFE, Polisulphones, Fluorates, ecc.) allowing a squashing of the sleeve on the needle, have drawbacks such as fragility and a not perfect accommodation (and consequently occlusion) to the corneal inlet cut. Furthermore, they are very expensive.

Sleeves realised by rigid materials (metals, glasses, quartz, ecc.) aims to avoid the contact, and therefore the friction, between the needle and the same sleeve, but it is very low their sealing of the corneal cut.

Furthermore, this kind of sleeve requires wider incisions with respect to the deformable sleeves.

Instead, studies on the needle aimed to minimise the above phenomena substantially concern modifications to the external geometry similar to those realised inside the sleeve (grooves or scoring), always in order to prevent or reduce the irrigation flow interruption.

Until now, it has not been suggested any solution aiming to reduce the needle friction coefficient, comprised of grade 5 titanium. The only solution suggested until now concerns the polishing of the needle in the portion that could be in contact with the silicon sleeve.

In this situation, it is well evident that a solution drastically reducing the friction coefficient of the needle would allow to obtain remarkable advantages in case of phacoemulsifier "via Pars-plana" intervention, wherein the crystalline is reached from the rear part of the bulb of the eye through the sclera, since such intervention provides the use of longer and thinner needles without irrigation sleeve.

In view of the above, the Applicant has realised a solution able to solve all the mentioned drawbacks of the solution known in the art.

Main object of the present invention is that of realising a particular set of needles to be used with crushing and sucking ultrasonic oscillating devices for different materials (organic and inorganic), in all those cases in which the needles contacts structures to be preserved.

It is therefore specific object of the present invention an improved tip for surgery, particularly for oculistic surgery, and more particularly for the excision of cataract, characterised in that the portion introduced within the patient's body is lined by PTFE (teflon).

In a preferred embodiment of the tip according to the invention, said tip has a junction proximal end and a distal end contacting the crystalline during the surgical intervention, and is characterised in that the portion between the distal end and the proximal end is lined by PTFE (teflon).

According to a preferred embodiment of the tip according to the invention, said distal end is subjected to an antireflection treatment.

Preferably, according to the invention, said junction proximal end is subjected to polishing.

Always according to the invention, said antireflection treatment on said distal end is carried out by sanding.

The portion of distal end upon which the antireflex treatment is applied, is preferably long between 1.5 and 2.5 mm.

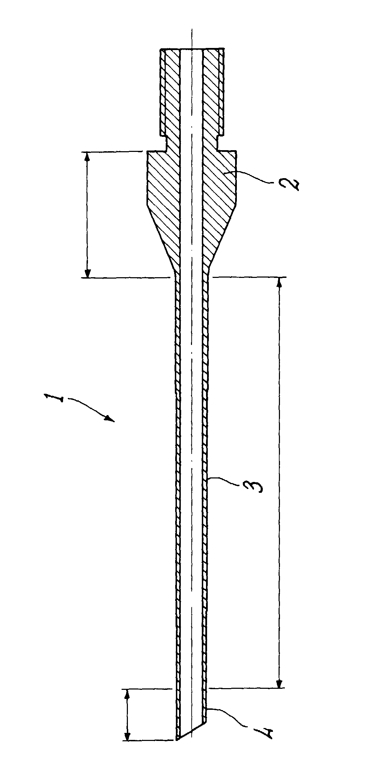

The present invention will be now described, for illustrative but not limitative purposes, according to its preferred embodiments, with particular reference to the figure of the enclosed drawing showing a longitudinal section of a tip according to the invention.

As it can be noted from the figure, a tip or needle 1 according to the invention can be ideally divided in three zones, respectively, observing from the right to left in the figure, a first zone 2 having a greater thickness, a second zone 3 of the needle and a third zone or end portion of the needle.

By the solution according to the invention it is suggested a particularly set of needles or tips 1 to be used with crashing and sucking ultrasonic oscillating devices for different materials (organic and inorganic), in all those case wherein the needle 1 contacts structures to be preserved.

The solution according to the invention particularly provides needles comprised of titanium alloy, the portion 3 of said needles which potentially can be in direct or indirect (by the sleeve) contact with organic structures (cornea, sclera, ecc.) to be preserved, is lined by PTFE (teflon).

PTFE lining must only be present on the portion 3 of the possible contact.

In fact, the distal part 4 of the needle 1, i.e. the portion contacting the crystalline to be crushed, is subjected to remarkable stresses, either of the mechanical type of for cavitation, which could rapidly lead to the separation of PTFE.

Furthermore, since said end 4 projects from the sleeve and is in the surgery visual field, it is useful that it is realised by an antireflex surface allowing to much more easily control the intervention.

For these reasons, according to the invention, the outer surface of the needle 1 along its distal portion 4 (1.5 - 2.5 mm) is preserved by the PTFE lining and suitably realised antireflex by a "sanding" treatment.

On the opposite side of the needle, the lining is interrupted close to the junction conical portion 2, since it is subjected to cavitation.

The so much evident cavitation stress on this portion 2 of the needle 1 (component of the oscillation perpendicular to the needle surface) leads to carry out a more suitable polishing treatment instead of the teflon lining that would be easily removed.

Therefore, by the solution according to the invention, a needle 1 is obtained for the excision of the crystalline by "phacoemulsifier", providing an outer surface having the features shown in the drawing, with a polished junction conical portion 2, a portion 3 lined by teflon and an end portion subjected to an antireflex treatment.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Improved tip for surgery, particularly for oculistic surgery, and more particularly for the excision of cataract, characterised in that the portion introduced within the patient's body is lined by PTFE (teflon).

2. Improved tip, according to claim 1 having a junction proximal end and a distal end contacting the crystalline during the surgical intervention, characterised in that the portion between the distal end and the proximal end is lined by PTFE (teflon).

3. Improved tip according to claim 2, characterised in that said distal end is subjected to an antireflection treatment.

4. Improved tip according to claim 2 or 3, characterised in that said junction proximal end is subjected to polishing.

5. Improved tip according to one of claims 3 - 4, characterised in that said antireflection treatment on said distal end is carried out by sanding.

6. Improved tip according to one of claims 3 - 5, characterised in that the portion of distal end upon which the antireflex treatment is applied, is long between 1.5 and 2.5 mm.
